# EUROPEAN PATENT APPLICATION

(11) **EP 3 763 392 A1**
(43) Date of publication of application: **13.01.2021**
(21) Application number: 20184431.3
(22) Date of filing: 07.07.2020
(51) Int. Cl.: A61K 47/69

(54) **LIPOSOME COMPOSITION WITH ADIPOCYTE SPECIFICITY AND PREPARATION PROCESS THEREOF**

(30) Priority: 08.07.2019 US 201962871336 P
(71) Applicant: TCI Co., Ltd., Taipei 11494 (TW)
(72) Inventor: LIN, Yung-Hsiang, 11494 Taipei (TW); CHANG, Jung, 11494 Taipei (TW)
(74) Representative: Viering, Jentschura & Partner mbB Patent- und Rechtsanwälte

(57) **Abstract**

A liposome composition with adipocyte specificity comprises a shell formed by a liposome material, and a ligand material bound to a surface of the shell. The ligand material is a plurality of long chain fatty acids (11), and the liposome composition recognizes and binds to an adipocyte (21) by the plurality of long chain fatty acids (11).

## Description

### BACKGROUND

### Technical Field

The present disclosure relates to a liposome composition and a preparation method thereof, and in particular to a liposome composition with adipocyte specificity and a preparation process thereof.

### Related Art

Fat is an essential component of the human body, but excessive fat content may damage the human body. In countries of which national economies increasingly take off, the problem of obesity only becomes more serious. Hence, in Asia, China will be one of the countries where the problem of obesity sharply rises in the next round.

In addition, Taiwan has become the fattest country among Asia at present. Among inhabitants of 18 years old or older, overweight ratios of the male and the female are 51.1 % and 35.8% respectively and still keep rising. Even the average age of an overweight age group is declining year by year, and an overweight ratio of primary and secondary school students has already risen to 25% or higher, which makes Taiwan the eighth highest region in the world. Therefore, obesity has been a problem urgently required to be solved at present.

However, current products used for losing fat or promoting lipolysis are mostly made from chemical components, and long-term use of these products is usually harmful to human health. Moreover, these products are usually expensive and not affordable for common users. To solve the problem above, those skilled in the art urgently need to develop a novel pharmaceutical product capable of improving lipolysis so as to realize a fat losing effect, and bring benefits to a broad group of people with such needs.

In addition, a liposome is mainly made from phospholipids and is a spherical vesicle formed by a lipid bilayer structure including an exposed hydrophilic layer and an inner hydrophobic layer. Since the liposome has a lipid structure similar to that of a cell membrane of an organism, the liposome has good biocompatibility and biodegradability, and can be widely used as a carrier for drug administering and releasing or for relevant component delivering. Administering an effective component by enclosing the effective component in a liposome carrier has the advantages such as increasing stability of the active component, reducing the toxicity of the active component, being suitable for delivery of various components, and the like. Furthermore, by means of controlling the lipid features and sizes, the probability of allergy can be reduced, the body metabolism can be accelerated, and the penetrability of drugs or the effective component can be enhanced as well.

However, a traditional liposome has the disadvantages of poor target specificity and insufficient stability. In addition, components in the liposome are prone to leaking out due to fracture of the liposome. As a result, the effects of delivering and acting of the liposome are greatly reduced. Therefore, how to provide a liposome composition with high target specificity is also an important topic in the art.

### SUMMARY

For the purpose aforementioned, one objective of the present disclosure is to provide a liposome composition with adipocyte specificity. The liposome composition includes a shell formed by a liposome material, and a ligand material bound to a surface of the shell. The ligand material is a plurality of long chain fatty acids, and the liposome composition recognizes and binds to an adipocyte by the plurality of long chain fatty acids.

In one embodiment of the present disclosure, the plurality of long chain fatty acids include at least one selected from a group consisting of a docosahexaenoic acid (DHA), an eicosapentaenoic acid (EPA), and a ω-3 fatty acid.

In one embodiment of the present disclosure, the plurality of long chain fatty acids are bound to the shell by a hydrophilic group, and the plurality of long chain fatty acids are bound to the adipocyte by a hydrophobic group.

In one embodiment of the present disclosure, the liposome composition with the adipocyte specificity further includes a core material. The core material is enclosed in the shell, and the core material is a material with a capability to promote fat metabolism and lipolysis.

In one embodiment of the present disclosure, the liposome material includes a phospholipid, and a weight ratio of phosphatidyl ethanol amine to phosphatidylcholine contained in the phospholipid is 3-6:1.

In one embodiment of the present disclosure, the weight ratio of the phosphatidyl ethanol amine to the phosphatidylcholine is 4:1.

In one embodiment of the present disclosure, with the liposome composition being 100 wt%, the phospholipid accounts for 0.5 wt% to 1.5 wt%.

In one embodiment of the present disclosure, with the liposome composition being 100 wt%, the ligand material accounts for 0.01 wt% to 0.06 wt%.

In one embodiment of the present disclosure, with the liposome composition being 100 wt%, the core material accounts for 0.1 wt% to 0.6 wt%.

In one embodiment of the present disclosure, the core material includes at least one selected from a group consisting of caffeine, vitamin C, vitamin E, vitamin B6, iron, magnesium, zinc, potassium, L-carnitine, a chlorogenic acid, chitin and garcinia cambogia.

Another objective of the present disclosure is to provide a method of preparing a liposome composition with adipocyte specificity. The method includes the following steps: (a) providing a shell formed by a liposome material; and (b) binding a ligand material to a surface of the shell. The ligand material is a plurality of long chain fatty acids, and the ligand material recognizes and binds to an adipocyte by means of the plurality of long chain fatty acids.

In conclusion, the liposome composition with the adipocyte specificity of the present disclosure has the effects of specifically targeting a specific kind of cell, and has the capability to promote the lipolysis, thereby achieving the effect of fat losing.

The implementations of the invention will be further described below. The embodiments listed below are used to describe the invention instead of limiting the scope of the invention. A person skilled in the art may make variations and modifications without departing from the spirit and scope of the invention. Therefore, the protection scope of the invention should be subject to the appended claims.

### BRIEF DESCRIPTION OF THE DRAWINGS

FIG. 1 is a schematic structure diagram of a liposome composition with adipocyte specificity of the present disclosure;
FIG. 2A is a schematic diagram showing the effect of the liposome composition with the adipocyte specificity of the present disclosure on targeting an OP9-derived adipocyte;
FIG. 2B is a schematic diagram showing the effect of the liposome composition with the adipocyte specificity of the present disclosure on targeting a mouse skeletal muscle cell C2C12;
FIG. 2C is a schematic diagram showing the difficulty for the liposome composition with the adipocyte specificity of the present disclosure to enter a human dermal fibroblast CCD966sk;
FIG. 3A is a schematic diagram showing the effect of the liposome composition with the adipocyte specificity of the present disclosure on promoting lipolysis;
FIG. 3B is a data diagram showing the effect of the liposome composition with the adipocyte specificity of the present disclosure on promoting lipolysis, in which * represents p<0.05, and ** represents p<0.01;
FIGS. 4A to 4B are components contained in lecithin used for preparing the liposome composition of Embodiment 1 of the present disclosure, wherein FIG. 4A is a chemical structural formula of phosphatidylcholine, and FIG. 4B is a chemical structural formula of phosphatidyl ethanol amine;
FIG. 4C is the ¹H-NMR spectrum of the lecithin in experimental group (the liposome composition of Embodiment 1); and
FIG. 4D is the ¹H-NMR spectrum of the lecithin in comparative group.

### DETAILED DESCRIPTION

### Definition

Numerical values used herein are approximate values, and all experimental data are within a range of ±20%, preferably within a range of ±10% and best within a range of ±5%.

Statistical analysis is conducted by using Excel. Data are expressed as average values ± standard deviation (STDEV), and the difference between each group is analyzed by the student's t-tests.

### Embodiment 1. Preparation of liposome composition with adipocyte specificity

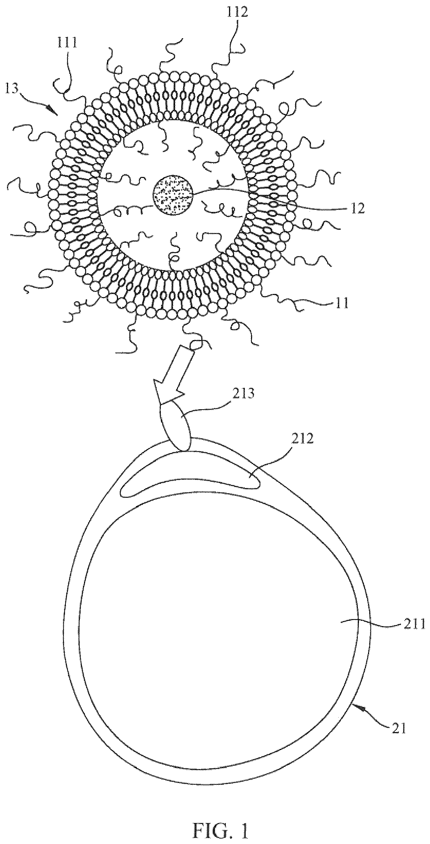

Firstly, components with proper quantities for preparing the liposome composition are prepared, and the components include a liposome material, an antioxidant, a ligand material, a core material, a vegetable gum, and a solution. The liposome material, such as a phospholipid (preferably lecithin, for example, soybean lecithin), is a structural material capable of forming a lipid monolayer or a lipid bilayer of a liposome. The antioxidant includes one or any combination of vitamin C, vitamin E (namely tocopherol) and tea polyphenols. Referring to FIG. 1, the ligand material is a material for recognizing and binding to a target, and long chain fatty acids (LCFA) 11 are used as the ligand material for recognizing an adipocyte in the present disclosure. The long chain fatty acids 11 are fatty acids with a carbon number greater than 12. For example, the long chain fatty acids 11 may include ω-3 fatty acid, docosahexaenoic acid (DHA) 111, eicosapentaenoic acid (EPA) 112, or any combination thereof. The core material 12 is a material enclosed inside the liposome 13, and can be released after the liposome 13 enters into the target. In a preferred embodiment of the present disclosure, the core material 12 includes at least one material with a capability to promote lipolysis, such as caffeine, vitamin C, vitamin E, vitamin B6, iron, magnesium, zinc, potassium, L-carnitine, a chlorogenic acid, chitin and garcinia cambogia. The vegetable gum can be one or any combination of Arabic gum, xanthan gum and Guar gum. The solution can be water or other acceptable solution that can be used to suspend the materials and produce the liposome.

The core material 12 is the material with the capability to promote the lipolysis as mentioned above. The functions of some core materials 12 will be explained below. For example, caffeine can promote metabolism and improve fat combustion. Vitamin C can reduce a cortisol level, which is a stress hormone that is generated through strenuous exercise to enable a body to enter into a catabolic state to burn muscles. Vitamin E can promote the metabolism of human body, improve blood circulation, facilitate food digestion and absorption, clear waste in human bodies, and avoid toxins accumulation in intestines and stomachs. Vitamin B6 can be converted into an enzyme, which can help human bodies to absorb and digest amino acids and to utilize glycogen energy, and can further facilitate releasing of a growth hormone during exercise. Iron not only participates in synthesis of hemoglobin, cytochrome and various enzymes, but also stimulates activity of various enzymes such as coenzyme A. Thus, iron can promote hematopoiesis, energy metabolism, growth and development, and have bactericidal functions. Moreover, the blood circulation of waists and abdomens can also be effectively promoted by supplementing iron element. Magnesium promotes muscle growth, prevents muscle cramping, further eases indigestion, and helps fat combustion to generate heat/energy. Zinc helps synthesis of testosterone and insulin-like growth factor (IGF) in bodies, which are the two important hormones that can promote muscle growth and are the key to muscle building. In addition, zinc can promote the metabolism of human bodies, and facilitates clearing of cholesterol in bodies. Also, zinc deficiency can influence appetite and decrease a digestion function. Potassium is very important to body fluid equilibrium in muscles. Keeping the body fluid under a good equilibrium makes the muscles enter into an anabolism state more easily. In addition, potassium can make bodies not absorb waste and can help weight loss with proper intake amount. L-carnitine can promote fat combustion, enhance performance during bodybuilding, and improve recovery capability. Chlorogenic acid can reduce glucose absorption, lower blood sugar concentration, inhibit fat absorption, and indirectly promote triglyceride decomposition, thereby realizing the assisting effect on weight control. Chitin can form positively charged food fiber after reacting with gastric acid, and the food fiber can be combined with a negatively charged bile acid and then be eliminated out of the bodies. Therefore, the fat emulsification conducted by bile can be reduced, thereby inhibiting fat absorption of human bodies. Garcinia cambogia can compete against citric acid in a metabolic cycle in the body to reduce fatty acid synthesis and therefore realize the purpose of body fat reduction. In addition, vitamin C and vitamin E can be used as the antioxidant or the core material 12.

The components of the liposome composition with the adipocyte specificity of the present disclosure are shown in Table 1 below.

**Table 1**

| | Components | Weight ratio (wt%) |
|---|---|---|
| Liposome material | Lecithin | 0.5-1.5 |
| Antioxidant | Vitamin E | 0.1-0.3 |
| Core material | Caffeine | 0.1-0.6 |
| Ligand material | DHA | 0.01-0.06 |
| Vegetable gum | Arabic gum | 3-10 |
| | Xanthan gum | 0.1-0.6 |
| | Guar gum | 0.1-0.5 |
| Solution | water | 81-91 |

The components shown in Table 1 are mixed uniformly to obtain a mixed solution. Then, the mixed solution is subjected to general homogenization for 10 min at 3,000 rpm (revolutions per minute), and then subjected to high-pressure homogenization at 800 bar for one cycle to obtain the liposome composition with the adipocyte specificity of the present disclosure.

As shown in FIG. 1, the ligand material can be embedded to the surface of the liposome 13. In a preferred embodiment, when the ligand material includes the long chain fatty acids 11, the hydrophilic group of the long chain fatty acids 11 can be embedded to the surface of the liposome 13, so that the hydrophobic group faces outwards. The hydrophobic group of the long chain fatty acids 11 is used as an anchor for recognizing and targeting (as shown by an arrow) fatty acid transport protein (FATP) 213 of the adipocyte 21 (including a lipid droplet 211 and a nucleus 212). Since the FATP 213 can penetrate out from a cell membrane, the hydrophobic group can be bound to the FATP 213 so as to specifically release the core material 12 to the target cell.

### Embodiment 2. Specificity Effect evaluation of liposome composition with adipocyte specificity on targeting adipocyte

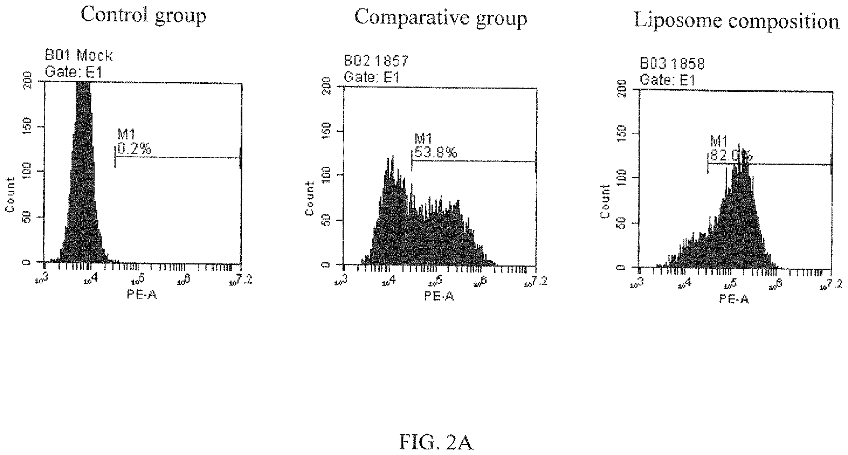

Firstly, mouse bone marrow stromal cells OP9 are prepared, which are purchased from American Type Culture Collection (USA ATCC®) with a serial number of CRL-2749™. 100,000 cells/2 mL of OP9 cells are inoculated in a culture tray, and the culture medium (minimum essential medium alpha medium (Gibco, USA), including 20 vol% fetal bovine serum (Gibco, USA) and 1 vol% of penicillin/streptomycin (Gibco, USA)) is changed every three days. The OP9 cells are differentiated into adipocytes after two weeks. In addition, human dermal fibroblasts CCD966sk are prepared, which are purchased from Food Industry Research and Development Institute with a serial number of BCRC No. 60153, and mouse skeletal muscle cells C2C12 are also prepared, which are purchased from USA ATCC® with a serial number of CRL-1772. 100,000 cells/2 mL of CCD966sk cells and 100,000 cells/2 mL of C2C12 cells are inoculated in the culture tray (different kinds of cell are inoculated in different well respectively) and cultured overnight. Then, the culture medium is replaced with 1.9 mL of a new culture medium in each well for the three kinds of cells (including the OP9, the CCD966sk and the C2C12), wherein the culture medium of the CCD966sk cells is a growth medium of a minimum essential medium in Earle's BSS including the 10 vol% of fetal bovine serum (purchased from Gibco); and a culture medium of the C2C12 cells is the Dulbecco's modified minimal essential medium (DMEM), in which Penicillin-streptomycin (Gibco) and fetal bovine serum (Gibco) are additionally added, so that the culture medium of the C2C12 cells includes 1 vol% of Penicillin-streptomycin (Gibco) and 10 vol% of fetal bovine serum (Gibco). For each kind of cells, one well is added with 100 µL of liposome compositions of Embodiment 1 (made by all the components recited in Table 1) and is cultured for 4 hours (the 100 µL of liposome compositions is dissolved in 2 mL of the corresponding culture medium to form a solution for being added, and so as below). Another well is added with 100 µL of liposome compositions (made by the components recited in Table 1 excluding the ligand material and the core material, which are replaced with water) to serve as a control group and is cultured for 4 hours. Still another well is added with 100 µL of liposome compositions (made by the components recited in Table 1 excluding the ligand material, which is replaced with water) to serve as a comparative group and is also cultured for 4 hours. Next, to each well of three kinds of cells, the culture medium is removed, then cleaning is conducted once with 1 mL of PBS, 0.2 mL of 1X trypsins are added, and culturing is conducted for 3 min at 37°C. Then, 0.8 mL of the corresponding culture medium is added to each well to neutralize the trypsins, and the culture medium and the cells of each well are collected into a 1.5 mL microcentrifuge tube. Next, centrifugation is conducted for 5 min at 400 g, a liquid supernatant is removed, then cleaning is conducted once with 1 mL of PBS, and then centrifugation is conducted for 5 min at 400 g. Then, a liquid supernatant is removed, and 200 µL of PBS is added to disperse the cells. Next, the cells are loaded on a flow cytometer for assaying fluorescence expression of the cells. It should be noted that when the preparation of the liposome is completed, a red fluorescent agent is added into the liposome composition. Particularly, a lipophilic membrane dye is added into every 1 ml of the liposome composition, and the lipophilic membrane dye can be bound to the lipid layer of the liposome to serve as a fluorophore during the assay of the flow cytometer. In the present disclosure, 0.05 mg/ml of Dil (1,1'-Dioctadecyl-3,3,3',3'-tetramethylindocarbocyanine perchlorate) is added into every 1 ml of the liposome composition. Results of each kinds of three cells are respectively shown in FIGS. 2A to 2C.

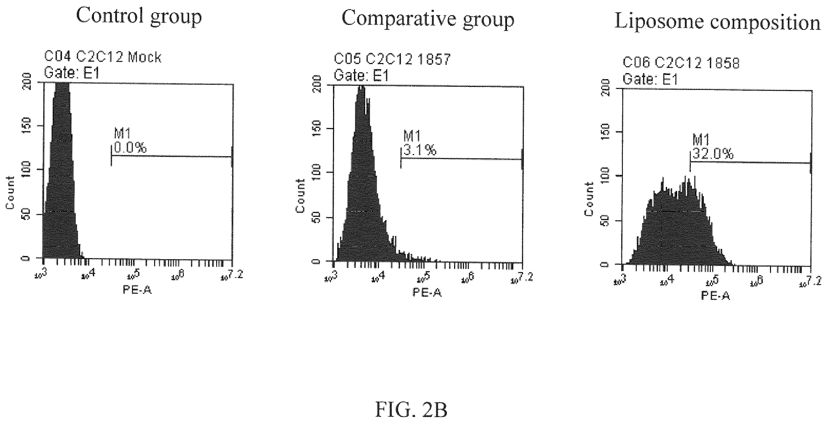

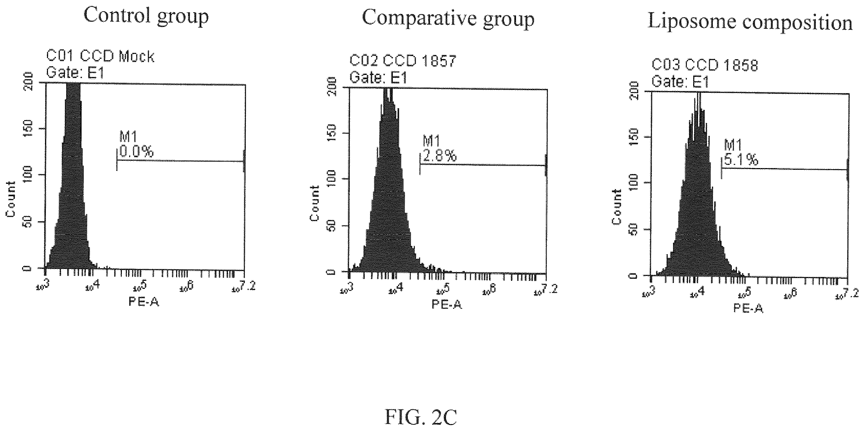

FIG. 2A is a schematic diagram showing the effect of the liposome composition with the adipocyte specificity of the present disclosure on targeting OP9-derived adipocytes. The OP9-derived adipocytes have high expression of fatty acid transport protein (FATP), and the ligand material contained in the liposome composition can recognize the FATP. FIG. 2B is a schematic diagram showing the effect of the liposome composition with the adipocyte specificity of the present disclosure on targeting mouse skeletal muscle cells C2C12, and the C2C12 cells have high FATP expression as well. FIG. 2C is a schematic diagram showing the difficulty for the liposome composition with the adipocyte specificity of the present disclosure to enter human dermal fibroblast cells CCD966sk, and the CCD966sk cells have low FATP expression. It can be seen from FIGS. 2A to 2C that the liposome composition with the adipocyte specificity of the present disclosure can specifically target the OP9-derived adipocyte and the mouse skeletal muscle cell C2C12, which are with high FATP expression, but cannot easily enter the human dermal fibroblast, which is with low FATP expression. In FIGS. 2A to 2C, the unit on Y axis is the cell number × fluorescence. It can be known from FIGS. 2A and 2B that, comparing to FIG. 2C, there are more liposomes (namely the liposome compositions of the Embodiment 1) entering the cells (the signals shown in the region M1 of FIGS. 2A and 2B are more than that of FIG. 2C). The results of the present embodiment verify that the liposome composition with the adipocyte specificity indeed has the effect of specifically targeting adipocytes.

### Embodiment 3. Effect evaluation of liposome composition with adipocyte specificity on promoting lipolysis

In the present embodiment, mouse bone marrow stromal cells are used for testing effect of the liposome composition with the adipocyte specificity of the present disclosure on promoting lipolysis. The mouse bone marrow stromal cells OP9 are purchased from American Type Culture Collection (USA ATCC®) with a serial number of CRL-2749™. The cells are cultured in a pre-adipocyte expansion medium before differentiation, the medium includes 79 vol% of minimum essential medium alpha medium (purchased from Gibco, USA, 12100-046), 20 vol% of fetal bovine serum (purchased from Gibco, USA), and 1 vol% of penicillin/streptomycin (purchased from Gibco, USA). A differentiation medium is used to differentiate the mouse bone marrow stromal cells, and the medium includes 79 vol% of the minimum essential medium alpha medium, 20 vol% of fetal bovine serum, and 1 vol% of penicillin/streptomycin .

To verify that the liposome composition with the adipocyte specificity of the present disclosure has the lipolysis promotion effect, firstly, the mouse bone marrow stromal cells are differentiated into adipocytes through a method that 8×10⁴ mouse bone marrow stromal cells OP9 and 500 µL of a pre-adipocyte expansion medium are inoculated in a 24-well culture tray, and cultured for 7 days at 37°C. Then, each well is replacement with a fresh differential medium every three days. Next, a microscope (ZEISS) is used for observing the forming of lipid droplets to guarantee that the cells are completely differentiated.

Then, the OP9 cells are divided into 4 groups, namely 1 control group, 2 comparative groups (including a comparative group 1 and a comparative group 2) and 1 experimental group. Cells belong to the comparative group 1 (caffeine group) are added with the materials recited in Table 1 except the liposome material (replaced with water), and the caffeine accounts for 0.5 wt%. Cells belong to the comparative group 2 (caffeine liposome group) are added with the materials recited in Table 1 except the ligand material (replaced with water), and the caffeine accounts for 0.5 wt%. Cells belong to the experimental group (caffeine liposome with ligands group) are added with all the materials recited in Table 1, and the caffeine accounts for 0.5 wt%. The cells in the control group are not subjected to any treatment. Next, the cells of each group are cultured for 7 to 10 days, and the culture medium is replaced every three days. Then, with reference to a user operation guide of a Glycerol cell-based assay kit of Cayman and the agents provided in the kit, glycerol assay is conducted on the cells of each group.

A liquid supernatant of the cell culture is collected from each well of the culture tray, and then 25 µL of the liquid supernatant of the cell culture and a glycerol standard are added into a new 96-well culture tray. Then, 100 µL of a reconstituted free glycerol assay reagent is added into each well, and then reaction is conducted at a room temperature for 15 min. Next, an ELISA reader (BioTek) is used for reading an OD_{540 nm} for obtaining the light absorption value of each group.

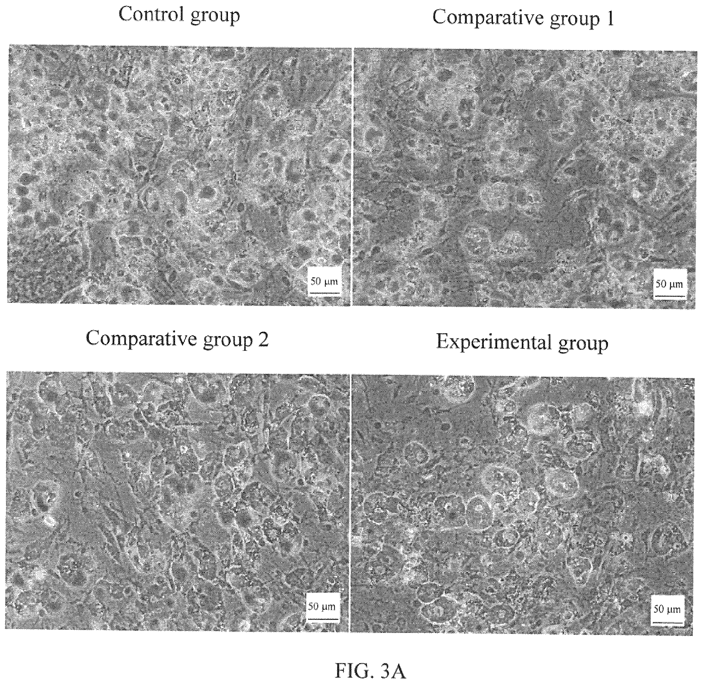

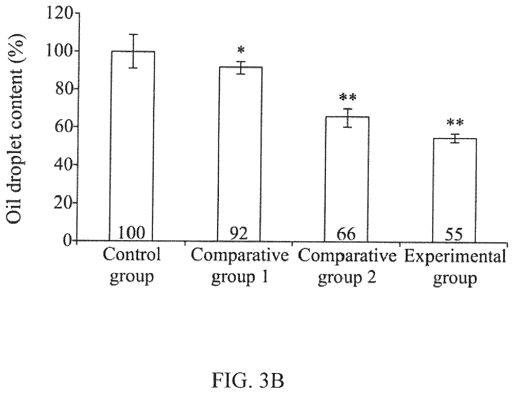

Results of the embodiment are shown in FIGS. 3A and 3B. FIG. 3A is a schematic diagram showing the effect of the liposome composition with the adipocyte specificity of the present disclosure on promoting lipolysis. FIG. 3B is a data diagram showing the effect of the liposome composition with the adipocyte specificity of the present disclosure on promoting lipolysis. It can be seen from FIGS. 3A and 3B that the oil droplet content of the experimental group is obviously lowered compared with that of the control group, the comparative group 1 and the comparative group 2. The oil droplet content of the experimental group is reduced by 45% and 11% respectively compared with that of the control group and the comparative group 2. The results of the present embodiment show that the liposome composition with the adipocyte specificity has the lipolysis promotion effect. Furthermore, since the ligand material of the liposome composition in Embodiment 1 can target the adipocyte, comparing to the liposome composition without the ligand material, the lipolysis promotion effect of the liposome composition in Embodiment 1 can be further improved.

### Embodiment 4. Effect of component ratio of lecithin on stability of the liposome

It is found that the liposomes formed by different lecithin may have different stability. For example, the liposome composition in Embodiment 1 (where the lecithin is purchased from Solae, LLC; product name SOLEC™ F) is much stable than the liposome formed by the same material in Table 1, except the lecithin is purchased from Lucas Neyer Cosmetics; product name EMULMETIK 900.

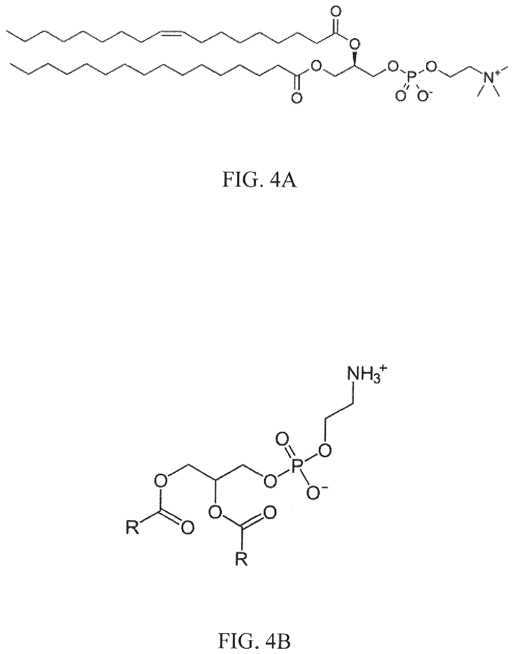

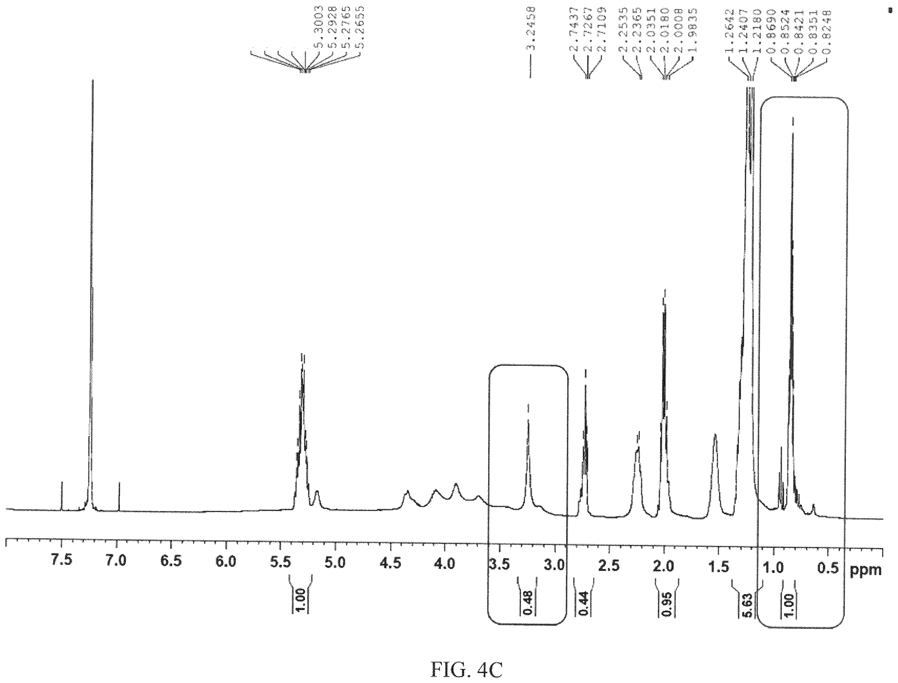

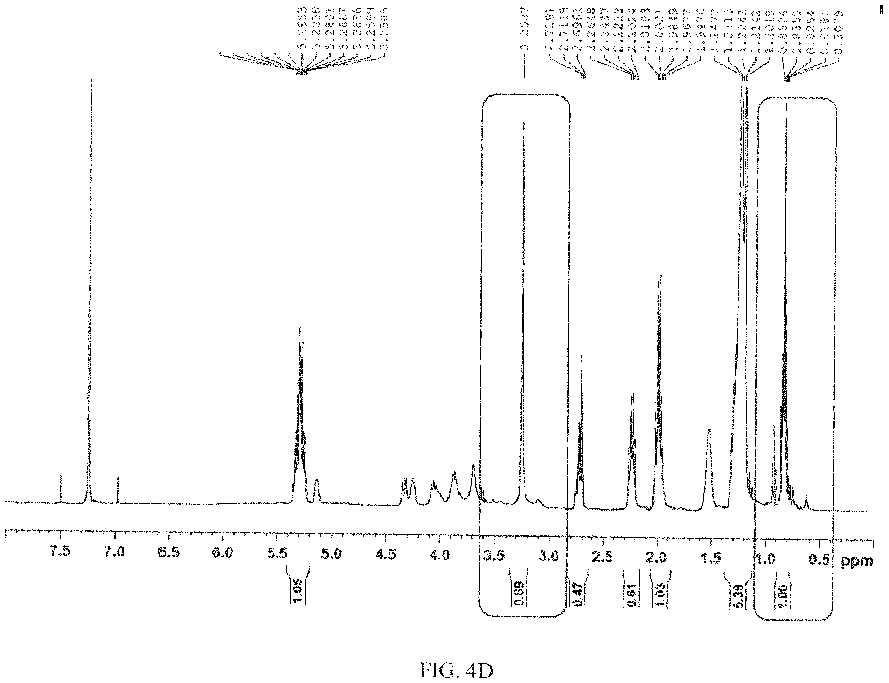

The lecithin (SOLEC™ F, purchased from Solae, LLC) used in Embodiment 1 and other lecithin available in the market (EMULMETIK 900, Lucas Neyer Cosmetics) are subjected to ¹H-NMR spectral comparison, and results thereof are shown in FIG. 4C and FIG. 4D respectively. FIG. 4A and FIG. 4B are chemical structural formulas of phosphatidylcholine and phosphatidyl ethanol amine contained in the lecithin. It can be known from FIG. 4C that the ratio of phosphatidyl ethanol amine to phosphatidylcholine contained in the lecithin used in Embodiment 1 is approximately 4:1, and the ratio of phosphatidyl ethanol amine to phosphatidylcholine contained in the lecithin in the comparative group is approximately 1:1 as shown in FIG. 4D. It can be speculated that instability of a structure of liposomes in the comparative group may be caused by large quantity of amido of the phosphatidylcholine.

In conclusion, the liposome composition with the adipocyte specificity of the present disclosure is capable of specifically targeting a specific cell (such as adipocytes and skeletal muscle cells), has the capability to promote lipolysis, and achieves a fat losing effect.

The foregoing descriptions are merely exemplary and not limitative. Any equivalent modifications or changes made without departing from the spirit and scope of the invention shall be included in the appended claims.

## Claims

1. A liposome composition with adipocyte specificity, **characterized in that** the liposome composition comprises:
a shell formed by a liposome material; and
a ligand material bound to a surface of the shell, wherein the ligand material is a plurality of long chain fatty acids (11), and the liposome composition is capable of recognizing and binding to an adipocyte (21) by the plurality of long chain fatty acids (11).

2. The liposome composition with the adipocyte specificity according to claim 1, **characterized in that** the plurality of long chain fatty acids (11) comprise at least one selected from a group consisting of a docosahexaenoic acid (DHA), an eicosapentaenoic acid (EPA), and a ω-3 fatty acid.

3. The liposome composition with the adipocyte specificity according to claim 1, **characterized in that** the plurality of long chain fatty acids (11) are bound to the shell by a hydrophilic group, and the plurality of long chain fatty acids (11) are bound to the adipocyte (21) by a hydrophobic group.

4. The liposome composition with the adipocyte specificity according to claim 1, **characterized in that** the liposome composition further comprising a core material (12), wherein the core material (12) is enclosed in the shell, and the core material (12) is a material with a capability to promote fat metabolism and lipolysis.

5. The liposome composition with the adipocyte specificity according to claim 1, **characterized in that** the liposome material comprises a phospholipid, and the phospholipid comprises a phosphatidyl ethanol amine and a phosphatidylcholine in a weight ratio of 3-6:1.

6. The liposome composition with the adipocyte specificity according to claim 5, **characterized in that** the weight ratio of the phosphatidyl ethanol amine to the phosphatidylcholine is 4:1.

7. The liposome composition with the adipocyte specificity according to claim 5, **characterized in that**, with the liposome composition being 100 wt%, the phospholipid accounts for 0.5 wt% to 1.5 wt%.

8. The liposome composition with the adipocyte specificity according to claim 1, **characterized in that**, with the liposome composition being 100 wt%, the ligand material accounts for 0.01 wt% to 0.06 wt%.

9. The liposome composition with the adipocyte specificity according to claim 4, **characterized in that**, with the liposome composition being 100 wt%, the core material accounts for 0.1 wt% to 0.6 wt%.

10. The liposome composition with the adipocyte specificity according to claim 9, **characterized in that** the core material (12) comprises at least one selected from a group consisting of caffeine, vitamin C, vitamin E, vitamin B6, iron, magnesium, zinc, potassium, L-carnitine, a chlorogenic acid, chitin and garcinia cambogia.
